# EUROPEAN PATENT APPLICATION

(11) **EP 4 721 749 A1**
(43) Date of publication of application: **08.04.2026**
(21) Application number: 24815135.9
(22) Date of filing: 10.05.2024
(51) Int. Cl.: A61K 35/32, A61P 43/00

(54) **CELL ACTIVATOR**

(30) Priority: 26.05.2023 JP 2023086930
(71) Applicant: U-Factor Co., Ltd., Tokyo 102-0085 (JP)
(72) Inventor: SHU, Yujing, Tokyo 102-0085 (JP); HORI, Keigo, Tokyo 102-0085 (JP); SETA, Yasuhiro, Tokyo 102-0085 (JP); OTAKE, Masato, Tokyo 102-0085 (JP); TERAMURA, Yuji, Tsukuba-shi, Ibaraki 305-8560 (JP); OHBA, Yoshio, Tsukuba-shi, Ibaraki 305-8560 (JP); KURAMOCHI, Akiko, Tsukuba-shi, Ibaraki 305-8560 (JP)
(74) Representative: HGF
(86) International application number: PCT/JP2024/017330
(87) International publication number: WO 2024/247651

(57) **Abstract**

Provided is a cell activator comprising a fraction isolated from a culture supernatant of a deciduous tooth pulp stem cell, wherein the fraction comprises no component of less than 3.5 kDa.

## Description

### TECHNICAL FIELD

The present invention relates to cell activators.

### BACKGROUND ART

Culture supernatants of mesenchymal stem cells (MSCs) are known to contain various growth factors and cytokines, and their usefulness is currently anticipated in a wide range of fields from beauty treatments to regenerative medicine. The MSCs can be readily collected from various tissues such as bone marrow, dental pulp, adipose tissues, and umbilical cords. In particular, deciduous teeth (baby teeth) pulp stem cells, which are MSCs that can be collected from deciduous teeth, have a potential to be particularly useful MSCs because the deciduous teeth pulp stem cells can be readily prepared from deciduous teeth, which have heretofore been discarded, and have high proliferative capacity.

Culture supernatants of deciduous teeth pulp stem cells have previously been reported to be useful in the repair of injured tissues of brain infarction or the like, or the protection of skin tissues (Patent Documents 1 and 2). However, it remains elusive what component in the culture supernatants is effective. Moreover, the culture supernatants may also contain components that act in an inhibitory manner on effective components, but such components are not yet known.

### REFERENCE DOCUMENT LIST

### PATENT DOCUMENTS

Patent Document 1: WO 2011/118795
Patent Document 2: WO 2014/126176

### SUMMARY OF THE INVENTION

### PROBLEM TO BE SOLVED BY THE INVENTION

The present invention aims to provide a composition with superior cell-activating effects by purifying a culture supernatant of baby teeth pulp stem cells.

### MEANS FOR SOLVING THE PROBLEM

The present inventors have earnestly conducted research and, as a result, identified a fraction having high cell-activating activity, and a fraction that inhibits cell-activating activity in a culture supernatant of deciduous tooth pulp stem cells, leading to the completion of the present invention.

Specifically, according to one embodiment, the present invention provides a cell activator comprising a fraction isolated from a culture supernatant of a deciduous tooth pulp stem cell, wherein the fraction comprises no component of less than 3.5 kDa.

According to one embodiment, the present invention provides use of a fraction isolated from a culture supernatant of a deciduous tooth pulp stem cell as a cell activator, wherein the fraction comprises no component of less than 3.5 kDa.

The fraction preferably comprises a component of 50 to 100 kDa and comprises no component of less than 30 kDa.

The fraction preferably comprises no component of more than 100 kDa and/or no component of 30 to 50 kDa.

The deciduous tooth pulp stem cell can be a primary cultured cell.

Alternatively, the deciduous tooth pulp stem cell can be an immortalized cell.

According to one embodiment, the present invention provides a method for activating a cell in a subject, comprising the step of administering the cell activator to the subject.

### EFFECTS OF THE INVENTION

The cell activator according to the present invention can be prepared readily and inexpensively from a culture supernatant of deciduous tooth pulp stem cells and comprises no component that inhibits cell-activating activity. Therefore, the cell activator is excellent in effect and safety and is useful.

### BRIEF DESCRIPTION OF THE DRAWINGS

FIG. 1 is a polyacrylamide gel electrophoresis image of SHED-CM and fractions thereof.
FIG. 2 is a polyacrylamide gel electrophoresis image of IM-SHED-CM and fractions thereof.
FIG. 3 is a graph showing results of a WST assay for evaluating the intracellular dehydrogenase activity of NIH3T3 cells with SHED-CM fractions having different fold concentrations.
FIG. 4 is a graph showing results of a WST assay for evaluating intracellular dehydrogenase activity of NIH3T3 cells cultured with IM-SHED-CM fractions having different fold concentrations.
FIG. 5 is a graph showing results of a WST assay for evaluating intracellular dehydrogenase activity of NIH3T3 cells cultured with a dilution of SHED-CM or a fraction thereof adjusted based on protein concentration.
FIG. 6 is a graph showing results of a WST assay for evaluating the intracellular dehydrogenase activity of NIH3T3 cells cultured with a dilution of IM-SHED-CM or a fraction thereof adjusted based on protein concentration.
FIG. 7 is a graph showing results of a WST assay for evaluating the intracellular dehydrogenase activity of NIH3T3 cells cultured with a dilution of a SHED-CM-derived 50-100 kDa fraction with a >100 kDa fraction or a 30-50 kDa fraction.
FIG. 8 is a graph showing results of a WST assay for evaluating the intracellular dehydrogenase activity of NIH3T3 cells cultured with a dilution of an IM-SHED-CM-derived 50-100 kDa fraction with a >100 kDa fraction or a 30-50 kDa fraction.
FIG. 9 is a diagram showing results of Western blot analysis for comparing the expression level and phosphorylation of MAPK in NIH3T3 cells cultured with SHED-CM/IM-SHED-CM or a fraction thereof.
FIG. 10 is a diagram showing results of Western blot analysis for comparing the expression level and phosphorylation of Akt in NIH3T3 cells cultured with SHED-CM/IM-SHED-CM or a fraction thereof.
FIG. 11 is a diagram showing results of Western blot analysis for comparing the expression level and phosphorylation of MAPK in NIH3T3 cells cultured with SHED-CM/IM-SHED-CM or a fraction thereof at the same protein concentration.
FIG. 12 is a diagram showing results of Western blot analysis for comparing the expression level and phosphorylation of Akt in NIH3T3 cells cultured with SHED-CM/IM-SHED-CM or a fraction thereof at the same protein concentration.
FIG. 13 is a diagram showing results of a scratch assay on NIH3T3 cells cultured with IM-SHED-CM or a fraction thereof.
FIG. 14 is a graph showing results of a WST assay for evaluating the intracellular dehydrogenase activity of NIH3T3 cells cultured with IM-SHED-CM or a >3.5 kDa fraction thereof.
FIG. 15 is a diagram showing results of a scratch assay on NIH3T3 cells cultured with IM-SHED-CM or a >3.5 kDa fraction thereof.
FIG. 16 is a graph showing results of a WST assay for evaluating the intracellular dehydrogenase activity of HUVECs cultured with a dilution of SHED-CM or a 50-100 kDa fraction thereof adjusted based on protein concentration.
FIG. 17 is a graph showing results of a WST assay for evaluating the intracellular dehydrogenase activity of HUVECs cultured with a dilution of IM-SHED-CM or a 50-100 kDa fraction thereof adjusted based on protein concentration.
FIG. 18 is a graph showing the intracellular dehydrogenase activity of HUVECs cultured with a 50-100 kDa fraction, or a mixture of > 100 kDa, 50-100 kDa, and 30-50 kDa fractions of SHED-CM.
FIG. 19 is a graph showing the intracellular dehydrogenase activity of HUVECs cultured with a 50-100 kDa fraction, or a mixture of 50-100 kDa and <30 kDa fractions of SHED-CM.
FIG. 20 is a graph showing the intracellular dehydrogenase activity of HUVECs cultured with a 50-100 kDa fraction, or a mixture of >100 kDa, 50-100 kDa, and 30-50 kDa fractions of IM-SHED-CM.
FIG. 21 is a graph showing the intracellular dehydrogenase activity of HUVECs cultured with a 50-100 kDa fraction, or a mixture of 50-100 kDa and <30 kDa fractions of IM-SHED-CM.
FIG. 22 is a graph showing results of a WST assay for evaluating the suppressive effect of IM-SHED-CM on cytotoxicity caused by hydrogen peroxide treatment.
FIG. 23 is a graph showing results of a WST assay for evaluating the suppressive effect of a 50-100 kDa fraction of IM-SHED-CM on cytotoxicity caused by hydrogen peroxide treatment.
FIG. 24 is a graph showing results of a WST assay for evaluating the suppressive effect of a >100 kDa fraction of IM-SHED-CM on cytotoxicity caused by hydrogen peroxide treatment.
FIG. 25 is a graph showing results of a WST assay for evaluating the suppressive effect of a 30-50 kDa fraction of IM-SHED-CM on cytotoxicity caused by hydrogen peroxide treatment.
FIG. 26 is a graph showing results of quantitatively analyzing the recovery of scratches in FIGs. 13 and 15.

### MODE FOR CARRYING OUT THE INVENTION

Hereinafter, the present invention will be described in detail. However, the present invention is not limited to embodiments described in the present specification.

According to a first embodiment, the present invention provides a cell activator comprising a fraction isolated from a culture supernatant of a deciduous tooth pulp stem cell, wherein the fraction comprises no component of less than 3.5 kDa.

In the present embodiment, the "cell activation" (and grammatical variations thereof) means the normalization or improvement of functions such as proliferation and metabolism of a cell. Thus, the "cell activation" according to the present embodiment may include the activation of enzymes related to cell proliferation and metabolism (various dehydrogenases, Akt, AMPK, etc.) or enzymes suppressing oxidative stress (superoxide dismutase (SOD), catalase (CAT), glutathione peroxidase (GPx), etc.). In this context, the "cell" can be of any vertebrate and is preferably a cell of a mammal such as a mouse, a rat, a rabbit, a pig, a bovine, a monkey, or a human, and is particularly preferably a human cell. The cell is not particularly limited by its type and can be, for example, a fibroblast, an endothelial cell, an epithelial cell, a neuron, a stem cell, a leukocyte, an osteocyte, a myocyte, or an adipocyte, preferably a fibroblast or an endothelial cell.

The "deciduous tooth pulp stem cell" is a mesenchymal stem cell collected from the pulp of a deciduous tooth. The deciduous tooth pulp stem cell according to the present embodiment can be derived from any vertebrate and is preferably derived from a mammal such as a mouse, a rat, a rabbit, a pig, a bovine, a monkey, or a human, is particularly preferably derived from a human.

Methods for preparing the deciduous tooth pulp stem cell have already been established (e.g., WO 2011/118795). The deciduous tooth pulp stem cell may be isolated and prepared from deciduous tooth pulp in accordance with methods known in the art.

The deciduous tooth pulp stem cell according to the present embodiment may be a primary cultured cell or may be an immortalized cell. The "immortalized cell" means that the cell, after repeating a given number of divisions, still maintains a state capable of proliferating, i.e., the cell has infinite proliferative capacity. A method for immortalizing cells has already been established, and a known approach can be adopted. Cells can be immortalized, for example, by introducing SV40T antigen gene or telomerase reverse transcriptase (TERT) gene to the cells.

Methods for culturing the deciduous tooth pulp stem cell have already been established (e.g., WO 2011/118795), and the deciduous tooth pulp stem cell can be cultured in accordance with methods known in the art. Specifically, the deciduous tooth pulp stem cells can be seeded at a density of, for example, 5×10⁴ to 2×10⁵ cells/mL, and cultured in a medium obtained, for example, by adding serum such as FBS or a serum replacement such as KSR, glucose, an amino acid, and a vitamin to a basal medium such as DMEM, IMDM, Ham's F-12, RPMI-1640, or a mixture thereof. The cell activator of the present embodiment is preferably prepared from a culture supernatant obtained by the deciduous tooth pulp stem cells cultured in a serum-free medium.

The cell activator of the present embodiment comprises no components of less than 3.5 kDa derived from the culture supernatant of deciduous tooth pulp stem cells. As used herein, the term "comprise no" means that the components to be removed are not detected by conventional detection methods (e.g., immunochemical methods). Thus, the cell activator of the present embodiment may be a material from which at least 85% by weight, 90% by weight or more, 95% by weight or more, 97% by weight or more, 98% by weight or more, or 99% by weight or more of the components of less than 3.5 kDa contained in the unpurified culture supernatant have been removed. In other words, the cell activator of the present embodiment may contain at most 15% by weight, less than 10% by weight, less than 5% by weight, less than 3% by weight, less than 2% by weight, or less than 1% by weight of the components of less than 3.5 kDa contained in the unpurified culture supernatant. The cell activator of the present embodiment exhibits an improved cell-activating effect as a result of removing the components of less than 3.5 kDa from the culture supernatant of deciduous tooth pulp stem cells.

The "kDa" according to the present embodiment can be calculated on the basis of, for example, SDS-PAGE or ultrafiltration. In the case of purifying the culture supernatant through a commercially available ultrafiltration filter unit, the "kDa" can be a value of a nominal molecular weight limit (NMWL) or a molecular weight cutoff (MWCO) indicated in the product.

The cell activator of the present embodiment preferably comprises a fraction comprising a component of 50 to 100 kDa, and preferably comprising no fraction comprising a component of less than 30 kDa. The cell activator of the present embodiment more preferably comprises no fraction comprising a component of more than 100 kDa and/or no fraction comprising a component of 30 to 50 kDa. This is because the component of 50 to 100 kDa has higher cell-activating activity, whereas not only the component of less than 3.5 kDa, but also the component of more than 100 kDa, and the component of 30 to 50 kDa might adversely affect the cell-activating activity of the component of 50 to 100 kDa.

The cell activator of the present embodiment may be prepared, for example, by using 100,000 MWCO, 50,000 MWCO, 30,000 MWCO, and 3,500 MWCO ultrafiltration membranes in combination. For example, a cell activator that comprises a component of 50 to 100 kDa and comprises no component of 30 to 50 kDa and no component of less than 30 kDa can be prepared from the culture supernatant of the deciduous tooth pulp stem cell by removing a component of more than 100 kDa from the culture supernatant through a 100,000 MWCO ultrafiltration membrane and concentrating the obtained filtrate through a 50,000 MWCO ultrafiltration membrane.

The cell activator of the present embodiment may be composed of only the fraction purified from the culture supernatant of the deciduous tooth pulp stem cell, but, in general, may further comprise a pharmaceutically acceptable carrier and additive as optional components.

The cell activator of the present embodiment can be prepared as a liquid agent or a solid agent and may be formulated in various dosage forms, for example, an injection, a jelly, a spray, a tablet, or granules. The cell activator of the present embodiment is preferably prepared as a liquid agent. In the case of preparing a liquid agent, sterilized water, physiological saline, an aqueous glucose solution, or the like can be used as a carrier, and a germicide, a tonicity agent, a stabilizer, or the like may be further blended therewith, if desired. In the case of preparing a solid agent, an additive such as starch, lactose, mannitol, or an inorganic salt and, if desired, a binder, a disintegrant, a lubricant, or the like can be blended therewith.

The content (based on a protein weight) of the component of 50 to 100 kDa derived from the culture supernatant of the deciduous tooth pulp stem cell in the cell activator of the present embodiment can be, for example, 60% by weight or more, 70% by weight or more, 80% by weight or more, or 90% by weight or more, and is preferably 60 to 70% by weight, when the total mass of the cell activator is defined as 100%.

The cell activator of the present embodiment comprises a component having cell-activating activity and comprises no component that inhibits it, among components derived from the culture supernatant of the deciduous tooth pulp stem cell. Hence, the cell activator of the present embodiment has a better cell-activating effect than that of the unpurified culture supernatant of the deciduous tooth pulp stem cell and is useful.

According to a second embodiment, the present invention provides a method for activating a cell in a subject, comprising the step of administering the cell activator to the subject. The terms "activating a cell" and "cell" according to the present embodiment are as defined in the first embodiment.

The "subject" according to the present embodiment can be any vertebrate and is preferably a mammal such as a mouse, a rat, a rabbit, a pig, a bovine, a monkey, or a human, and is particularly preferably a human. The subject can be of any age including an infant, a youngster, an adolescent, an adult, and elderly subjects.

The cell activator in the method of the present embodiment can be formulated in various dosage forms as defined in the first embodiment, and can be administered by any administration method suitable for each dosage form, for example, oral administration, intravenous administration, subcutaneous administration, percutaneous administration, intramuscular administration, transnasal administration, intraperitoneal administration, direct injection to a target tissue, or administration by inhalation. The dose of the cell activator can differ depending on the age, body weight, health condition, and the like of the subject and can be, for example, 0.1 to 10 mg/kg (body weight), preferably 0.5 to 2 mg/kg (body weight). The dose may be administered in a single dose or in multiple doses.

According to a third embodiment, the present invention provides use of a fraction isolated from a culture supernatant of a deciduous tooth pulp stem cell as a cell activator, wherein the fraction comprises a component of 50 to 100 kDa and comprises no component of less than 30 kDa. The terms "deciduous tooth pulp stem cell", "cell activator", "cell", "comprise no", and "kDa" according to the present embodiment are as defined in the first embodiment. A fraction derived from a culture supernatant of a deciduous tooth pulp stem cell, the fraction comprising a component of 50 to 100 kDa and comprising no component of less than 30 kDa, can be used as a cell activator in accordance with the contents described in the first and second embodiments.

### EXAMPLES

Hereinafter, the present invention will be further described with reference to Examples. These examples do not limit the present invention by any means.

### <Material and reagent>

NIH3T3 cells were purchased from JCRB Cell Bank. Human umbilical vein endothelial cells (HUVECs), EBM(TM)-2 basal medium (CC-3156), and EGM(TM)-2 supplement kit (CC-4176) were purchased from Lonza K.K. Dulbecco's modified Eagle medium (DMEM) was purchased from GIBCO. Bovine calf serum (hereinafter, referred to as "CS") and an antimycotic solution (100×) were purchased from Sigma-Aldrich Co. LLC. Fetal bovine serum (FBS) and a 0.05% trypsin/EDTA solution were purchased from Invitrogen Corp. Charcoal (powder, activated) (hereinafter, referred to as "activated charcoal") was purchased from Nacalai Tesque, Inc. A Dulbecco's phosphate buffer solution (PBS), a RIPA buffer, and a quick CBB staining liquid were purchased from FUJIFILM Wako Pure Chemical Corp. An ultrafiltration unit (Vivaspin Turbo 15 membrane 30,000, 50,000, 100,000 MWCO) was purchased from Sartorius AG. Millex-GV low protein binding Durapore (PVDF) Membrane 0.22 µm was purchased from Merck Millipore. Spectra/Por(TM) 7 Dialysis Membrane (MWCO: 3.5 kD) was purchased from Repligen Corp. A PD-10 column was purchased from Cytiva. Micro BCA Protein Assay Kit was purchased from Thermo Fischer Scientific Inc. Cell Counting Kit-8 was purchased from Dojindo Laboratories. Acrylamide, protein standard markers, and Clarity Western ECL Substrate were purchased from Bio-Rad Laboratories, Inc. Protease inhibitor complete (#11 697 498 001) was purchased from F. Hoffmann-La Roche Ltd. An anti-phospho p44/42 antibody (#9106) and an anti-phospho Akt antibody (#9271) were purchased from Cell Signaling Technology, Inc. An anti-mouse IgG-HRP antibody (#62-6520) and an anti-rabbit IgG-HRP antibody (#65-6120) were purchased from Invitrogen Corp.

### <1. Preparation of deciduous tooth pulp-derived stem cell culture supernatant and fractions thereof>

### (1-1) Preparation of stem cells from human exfoliated deciduous teeth (SHED)

Exfoliated or extracted human deciduous teeth were disinfected with a 5% chlorhexidine solution (Yamazen Corp.) or a 10% povidone-iodine (Iwaki Seiyaku Co., Ltd.). Then, the crown parts of the teeth were divided, and pulp tissues were recovered using a dental reamer. The pulp tissues were suspended in DMEM containing 10% (v/v) of FBS. 2 mg/ml collagenase (FUJIFILM Wako Pure Chemical Corp.) and Dispase (FUJIFILM Wako Pure Chemical Corp.) were added thereto, and the mixture was incubated at 37°C for 1 hour. A supernatant was removed by centrifugation at 777×g for 5 minutes to recover pulp cells. The pulp cells were suspended in DMEM (4 mL) supplemented with 10 % (v/v) of FBS and 1% (v/v) of antibiotic-antimycotic (Gibco^{™} Antibiotic-Antimycotic), and seeded in a 6-well plate. The cells were cultured under conditions of 37°C and 5% CO₂ until reaching subconfluency. A 0.05% trypsin/EDTA solution was added thereto, and the cells were incubated at 37°C for 5 minutes, recovered, and seeded in a 10 cm dish for an adhesive cell (Violamo). Then, the cells were subcultured three times and allowed to proliferate into approximately 1×10⁷ cells. A 0.05% trypsin/EDTA solution was added thereto, and the cells were incubated at 37°C for 5 minutes. The recovered cells were designated as "SHED (stem cells from human exfoliated deciduous teeth)".

### (1-2) Preparation of immortalized stem cells from human exfoliated deciduous teeth (IM-SHED)

SHED immortalized by the introduction of the SV40 gene using a viral vector (IM-SHED) was prepared under contract with Applied Biological Materials Inc.

### (1-3) Preparation of culture supernatant

SHED or IM-SHED prepared as described above was subcultured in DMEM containing 10% (v/v) of FBS. For the preparation of the culture supernatant, SHED within 8 passages and IM-SHED at passages 50 to 55 were used. The medium was replaced with serum-free DMEM, and the cells were cultured for 48 hours. Then, the medium was recovered and filtered through a separation membrane (Stericup Quick Release-GP, PVDF, 0.22 µm; Merck Millipore) to obtain a culture supernatant (conditioned medium: CM). Hereinafter, the SHED-derived culture supernatant is referred to as "SHED-CM", and the IM-SHED-derived culture supernatant is referred to as "IM-SHED-CM".

### (1-4) Fractionation and concentration of culture supernatant

CM was centrifuged at 1,000 rpm at room temperature for 3 minutes; at 500×g at 4°C for 30 minutes; and at 2,000×g at 4°C for 30 minutes and was filtered through Millex-GV low protein binding Durapore (PVDF) Membrane 0.22 µm to remove impurities such as cells. Then, CM was applied to a 100,000 MWCO ultrafiltration unit and centrifuged at 2,000×g at 4°C for 20 minutes. The filtrate (hereinafter, referred to as "PT") was applied to a 50,000 MWCO ultrafiltration unit and centrifuged at 2,000×g at 4°C for 20 minutes. The resulting PT was applied to a 30,000 MWCO ultrafiltration unit and centrifuged at 2,000×g at 4°C for 20 minutes.

10 mL of PBS was further added to the 100,000 MWCO ultrafiltration unit and centrifuged at 2,000×g at 4°C for 20 minutes. This operation was repeated five times to obtain a >100 kDa fraction concentrate. The PT obtained as described above was applied to the 50,000 MWCO ultrafiltration unit and was centrifuged at 2,000×g at 4°C for 20 minutes to obtain a 50-100 kDa fraction concentrate. The resulting PT was applied to the 30,000 MWCO ultrafiltration unit and was centrifuged at 2,000×g at 4°C for 20 minutes to obtain a 30-50 kDa fraction concentrate. The resulting PT was applied to a PD-10 column, and the buffer was replaced with PBS to obtain a <30 kDa fraction solution. Each fraction concentrate was freeze-dried and then dissolved in Milli-Q water, and a sample buffer (SDS-containing or SDS-free) was added to the solution, followed by electrophoresis on a 12.5% polyacrylamide gel.

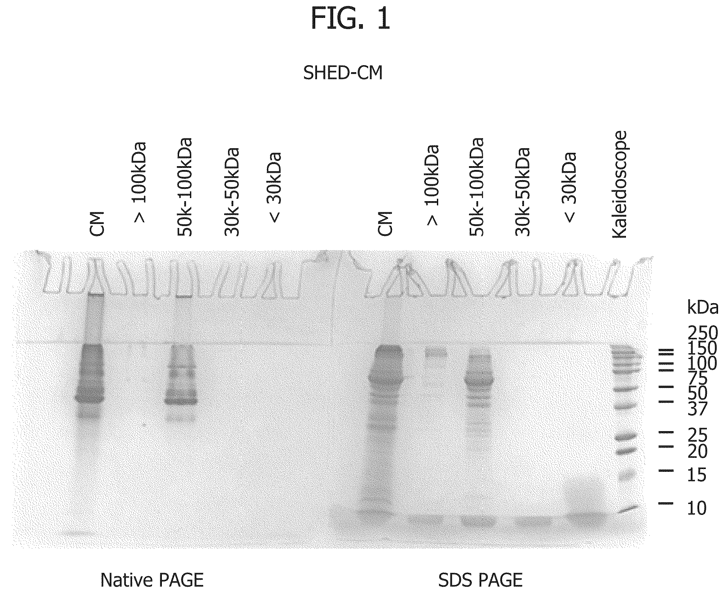

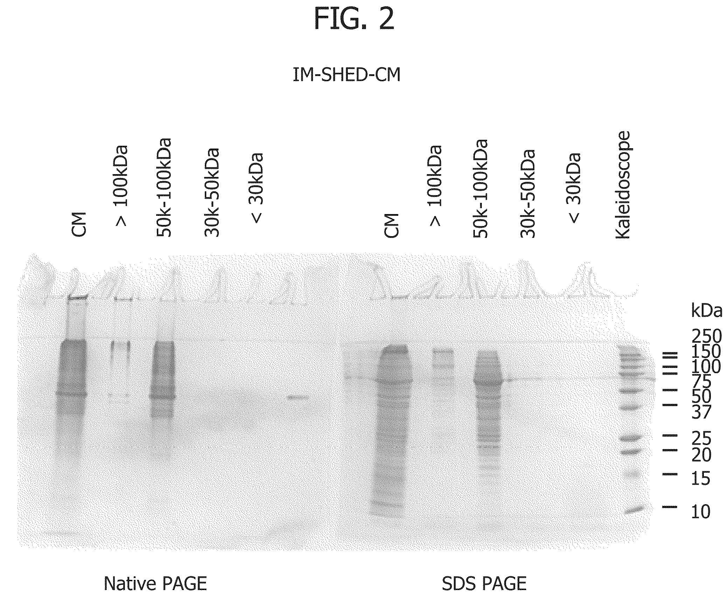

FIG. 1 shows electrophoresis results about SHED-CM and the fractions thereof. FIG. 2 shows electrophoresis results about IM-SHED-CM and the fractions thereof. Both SHED-CM and IM-SHED-CM were confirmed to contain an abundance of protein components in the 50-100 kDa fraction.

### <2. Activation of cell proliferation and viability by SHED-CM/IM-SHED-CM fraction (WST assay)>

Activated charcoal-treated CS (hereinafter, referred to as "CH-CS") was prepared by the following procedures: 10 mg of activated charcoal was added to 1 mL of CS, and the mixture was mildly vortexed for 15 seconds. After incubation at 37°C for 10 minutes, the resultant was centrifuged at 12,000 rpm at 4°C for 20 minutes, and a supernatant was applied to a 0.22 µm PVDF filter to obtain CH-CS. NIH3T3 cells were seeded (5,000 cells/well) in a 96-well plate (Violamo) and were cultured overnight in 10% CS/DMEM. In order to create a serum-starved state, the cells were washed once with DMEM, and the medium was then replaced with 0.4% CH-CS/DMEM, followed by culture for 24 hours. Then, each fraction prepared as described in section 1 above was diluted with 0.4% CH-CS/DMEM and added to the cells, which were then cultured for 44 hours. A WST solution (10 µL/well) of Cell Counting Kit-8 was added to the cells, which were then further cultured for 4 hours, followed by the measurement of absorbance at 450 nm.

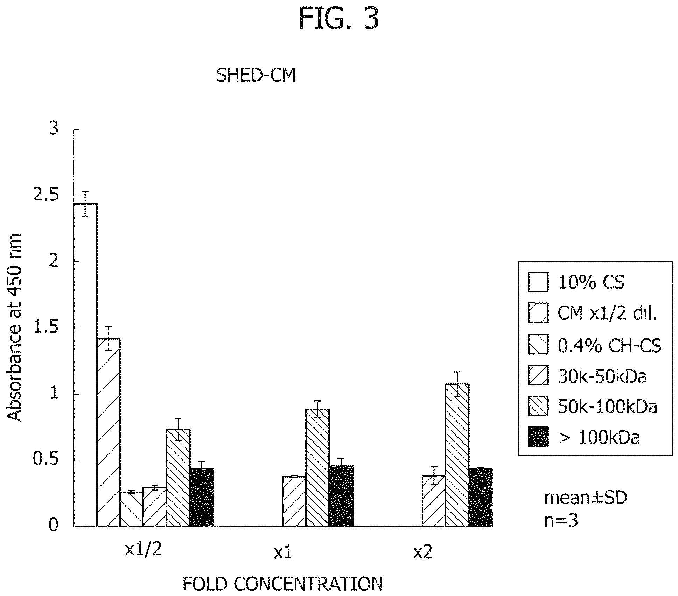

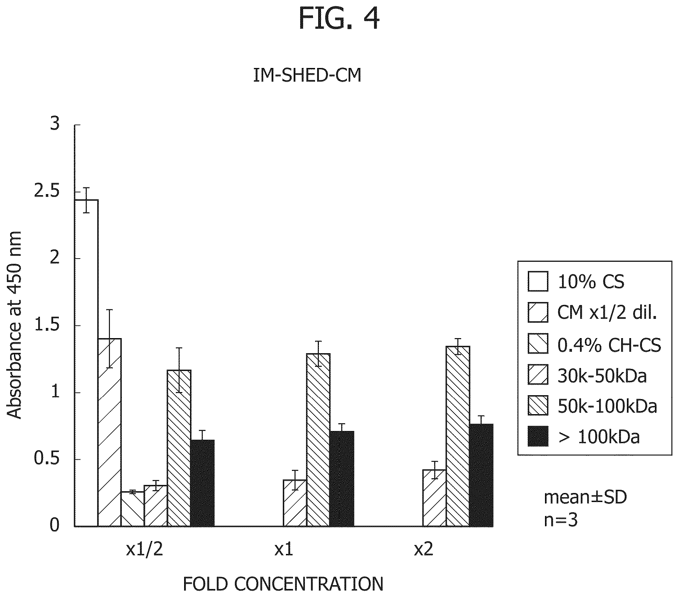

The results are shown in FIGs. 3 and 4. An increase in absorbance at 450 nm indicates an increase in activity of intracellular dehydrogenase, and higher absorbance at 450 nm is interpreted as higher proliferative capacity and viability of the cells. In the figures, "10% CS" denotes 10% CS/DMEM, and "CM×1/2 dil." denotes a CM medium diluted 1/2 with 0.4% CH-CS/DMEM. From these results, both SHED-CM and IM-SHED-CM were found to contain a component that activates cell proliferation and viability in the 50-100 kDa fraction. Similar results were also obtained for human uterine cervical cancer-derived cell line HeLa cells, human bone marrow-derived mesenchymal stem cells, and human iPS cells (409B2 line) (data not shown).

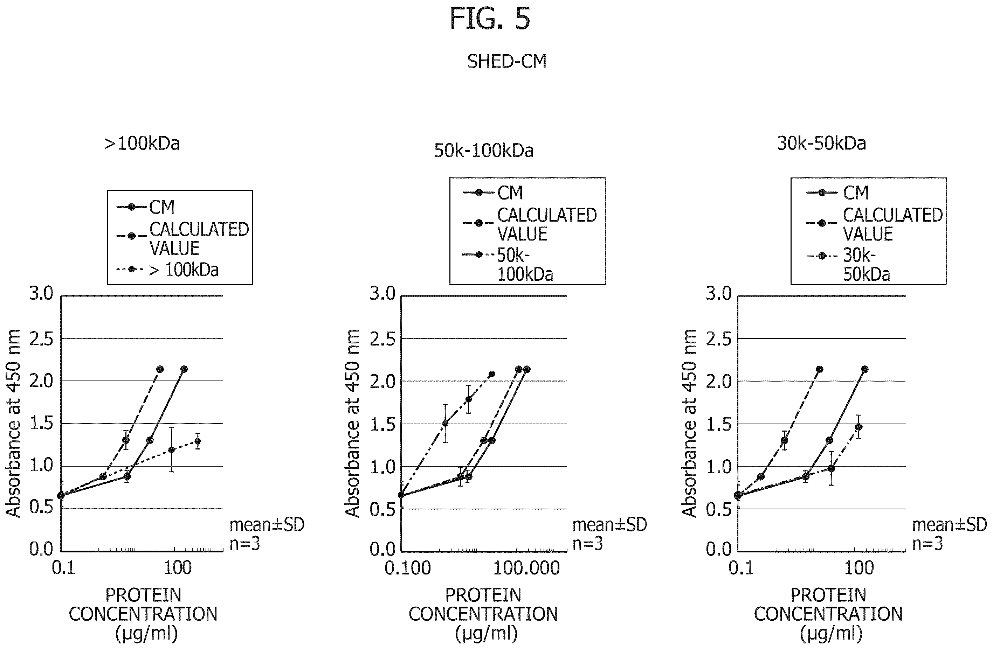

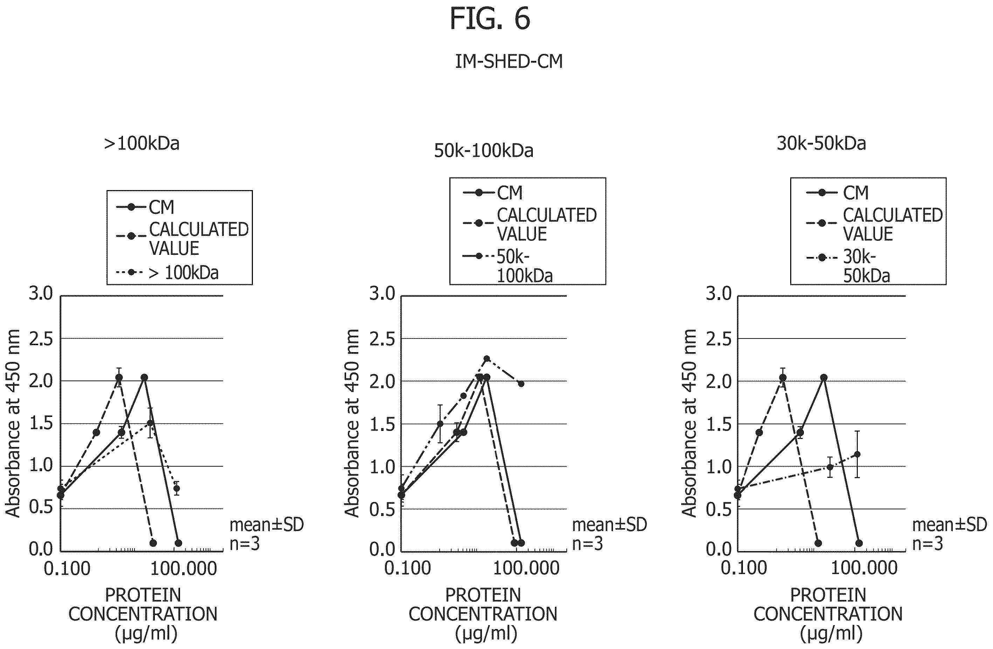

FIGs. 5 and 6 show results of comparing the activity of each fraction at the same protein concentration. In the figures, "calculated value" indicates a value obtained by correcting the CM results based on the protein concentration of each fraction, which was determined from the electrophoresis results described in section 1 above and protein quantification using the Micro BCA Protein Assay Kit. Among the fractions purified from SHED-CM and IM-SHED-CM, only the 50-100 kDa fractions were found to contain a component that activates cell proliferation and viability.

### <3. Inhibition of cell proliferation and viability by SHED-CM/IM-SHED-CM fraction>

A dilution of the 50-100 kDa fraction prepared as described in section 1 above with 0.4% CH-CS/DMEM, the >100 kDa fraction, or the 30-50 kDa fraction was prepared and evaluated for intracellular dehydrogenase activity by the same procedures as described in section 2 above.

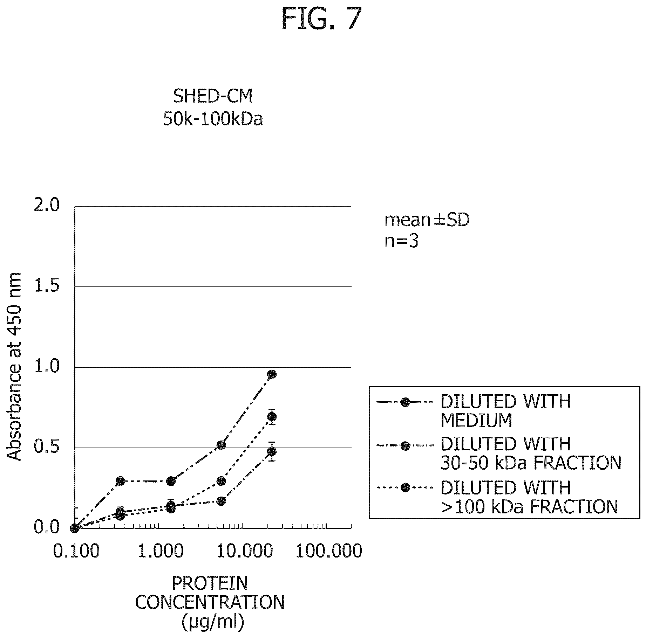

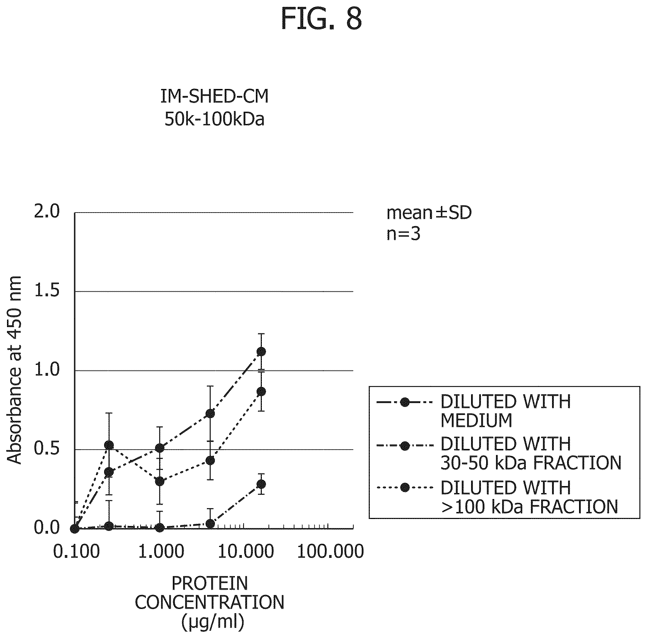

The results are shown in FIGs. 7 and 8. The 50-100 kDa fraction mixed with the >100 kDa fraction or the 30-50 kDa fraction reduced the activity of intracellular dehydrogenase. In particular, the IM-SHED-derived 30-50 kDa fraction had a large inhibitory effect (FIG. 8), suggesting the presence of a factor that inhibits the proliferative capacity and viability of the cells.

### <4. Activation of intracellular signal transduction by SHED-CM/IM-SHED-CM fraction>

NIH3T3 cells were seeded in a 12-well plate (6×10⁴ cells/well) and were cultured overnight in 10% CS/DMEM. In order to create a serum-starved state, the cells were washed once with DMEM, and the medium was then replaced with 0.4% CH-CS/DMEM, followed by culture for 24 hours. Then, CM or each fraction prepared as described in section 1 above was diluted with 0.4% CH-CS/DMEM and was added to the cells. 10% FBS or human TNFα (PeproTech, Inc.) (50 ng/mL) was added to a control. The cells were cultured for 30 minutes, then washed twice with PBS, and lysed with a RIPA buffer supplemented with a protease inhibitor. The cell lysate was recovered into a microtube and was shaken at 4°C for 30 minutes. A supernatant was recovered by centrifugation at 15,000 rpm at 4°C for 20 minutes. An SDS-containing sample buffer was added thereto, and the mixture was incubated at 95°C for 5 minutes, followed by electrophoresis on a 10% polyacrylamide gel. Proteins were transferred to a nitrocellulose membrane and then blocked with a 5% skim milk/TBST solution (room temperature, 1 hr). Then, the nitrocellulose membrane was incubated in a primary antibody solution overnight at 4°C. The primary antibody solution was removed, and the nitrocellulose membrane was washed three times with TBST and then was incubated in a secondary antibody solution at room temperature for 1 hour. The primary antibody used was an anti-phospho p44/42 antibody (1:2000) or an anti-phospho Akt antibody (1:2000), and the secondary antibody used was an anti-mouse IgG-HRP antibody (1:5000) or an anti-rabbit IgG-HRP antibody (1:5000). Clarity Western ECL Substrate was used in detection reaction.

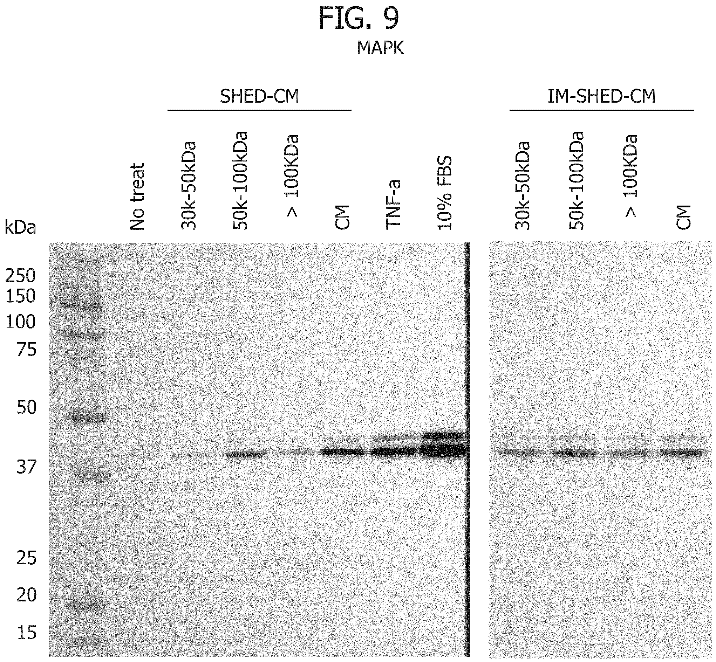

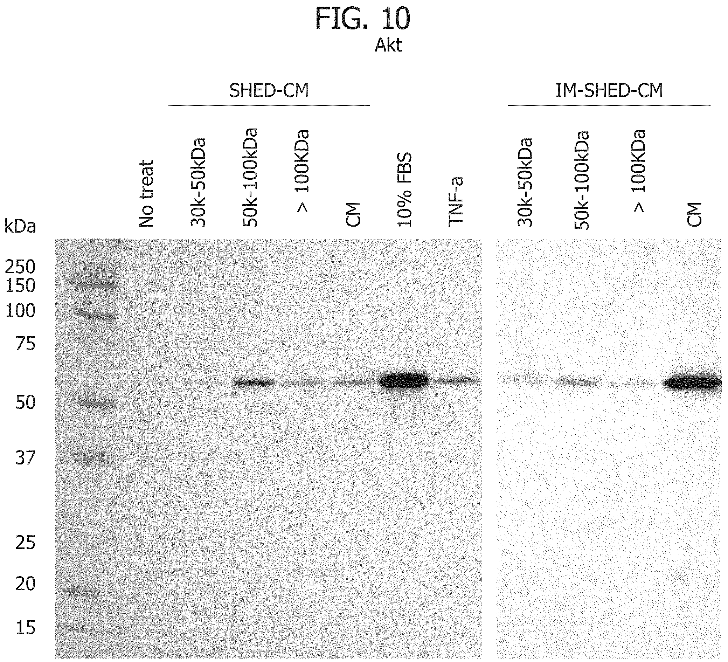

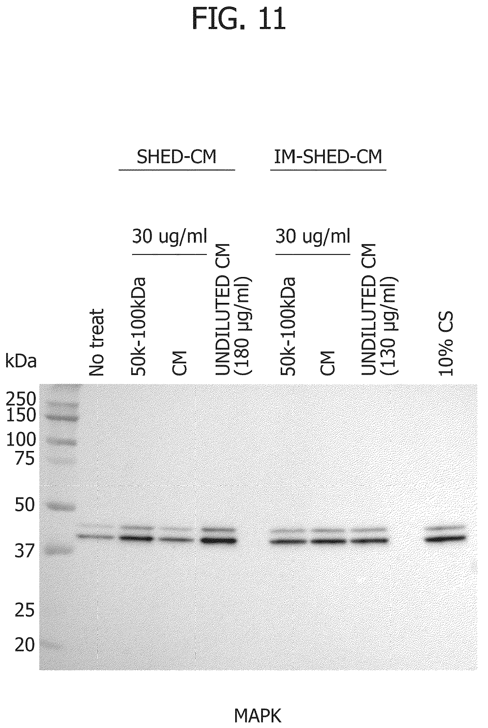

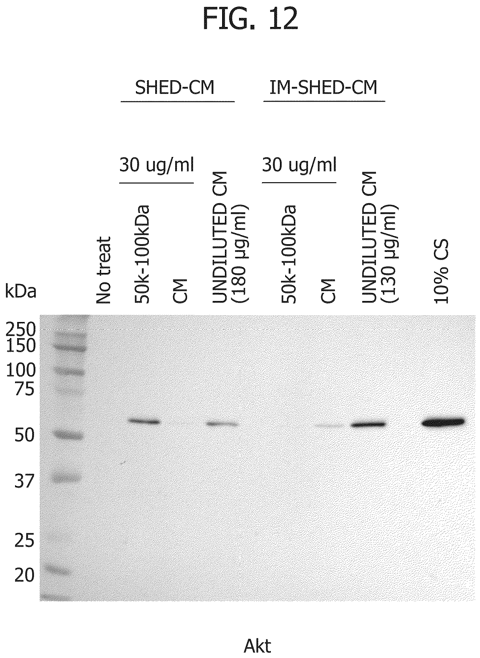

The results are shown in FIGs. 9 and 10. The addition of the 50-100 kDa fraction markedly increased the expression and phosphorylation of MAPK and Akt. FIGs. 11 and 12 show results comparing CM and the 50-100 kDa fraction adjusted to the same protein concentration. An increase in phosphorylation by the 50-100 kDa fraction compared to CM was observed. These results indicate that the 50-100 kDa fraction contains a component that activates intracellular phosphorylation signaling.

### <5. Activation of cell migration by SHED-CM/IM-SHED-CM fraction (scratch assay)>

NIH3T3 cells were seeded in a 48-well plate (3.75×10⁴ cells/well) and were cultured overnight in 10% CS/DMEM. In order to create a serum-starved state, the cells were washed once with DMEM, and the medium was then replaced with 0.4% CH-CS/DMEM, followed by culturing for 24 hours. The cell monolayer was scratched in a straight line using a 200 µL pipette tip to create a scratch. After washing twice with DMEM, and CM (diluted 1/2 with 0.4% CH-CS/DMEM); 10% FBS/DMEM; or each of the >100 kDa, 50-100 kDa, and 30-50 kDa fractions (diluted with 0.4% CH-CS/DMEM to a protein concentration 5-fold higher than that of the original CM) was added, and the cells were cultured for 24 hours.

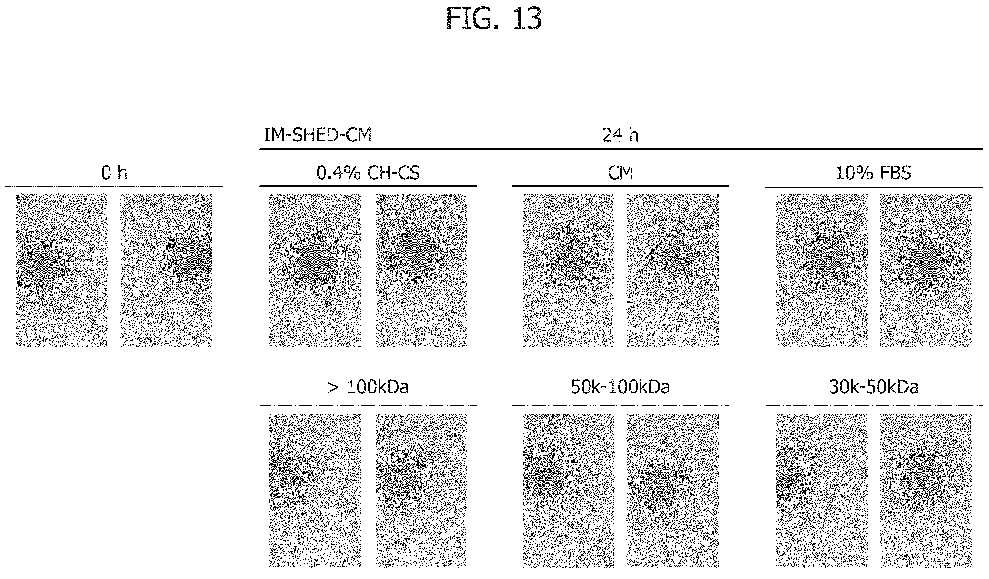

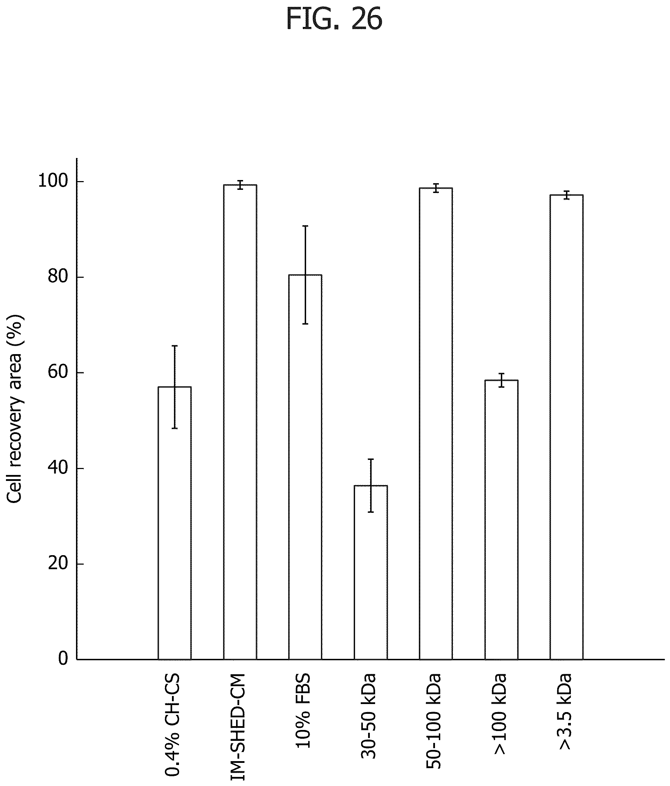

The results (microscopic images) are shown in FIG. 13. The cell area in the microscopic images was converted to a numeric value using ImageJ software and quantitatively analyzed. The results are shown in FIG. 26. The 50-100 kDa fraction was confirmed to retain wound-healing activity equivalent to that of CM.

### <6. Preparation and analysis of ≥3.5 kDa fraction of IM-SHED-CM>

IM-SHED-CM was placed in Spectra/Por^{™} 7 Dialysis Membrane (MWCO: 3.5 kDa) and dialyzed against a 100-fold volume of Milli-Q water for 72 hours. During this period, Milli-Q water was changed eight times. The dialyzed solution was freeze-dried, dissolved in PBS at 1/10 of the starting volume (on ice, >30 min), and filtered through a 0.22 µm PVDF filter. The obtained ≥3.5 kDa fraction was evaluated for its activity against intracellular dehydrogenase and cell migration by the same procedures described in sections 2 and 5 above.

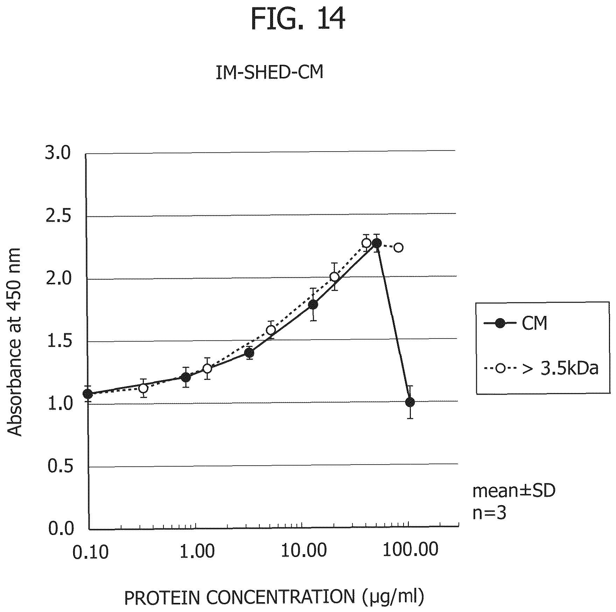

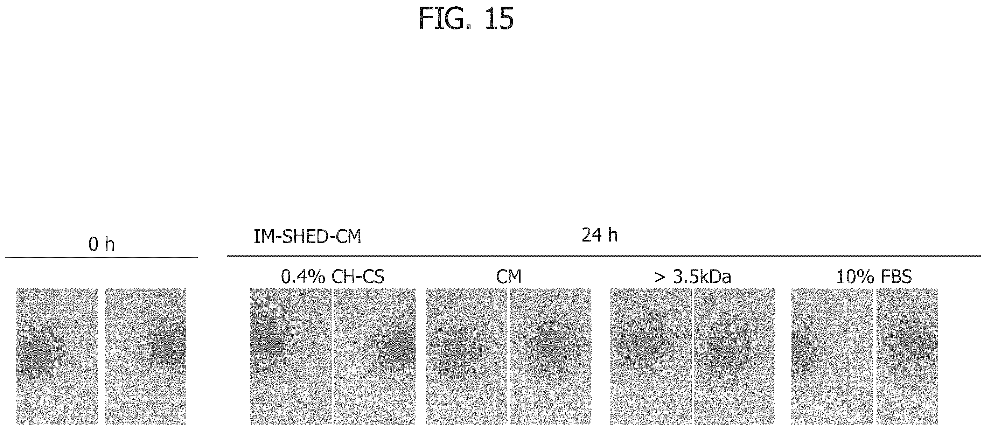

The results of the WST assay are shown in FIG. 14. The ≥3.5 kDa fraction adjusted to a high protein concentration (100 µg/mL) showed greater activation of intracellular dehydrogenase than CM adjusted to the same protein concentration. The results of the scratch assay are shown in FIG. 15, and results of the quantitative analysis are shown in FIG. 26. The ≥3.5 kDa fraction markedly increased cell migration into the scratch. These results demonstrate that removal of the <3.5 kDa fraction yields a cell-activating effect equivalent to or superior to that of CM.

### <7. Activation of cell proliferation and viability by SHED-CM/IM-SHED-CM fraction - (2)>

The activation of intracellular dehydrogenase by a 50-100 kDa fraction purified from SHED-CM or IM-SHED-CM was evaluated using HUVECs instead of NIH3T3 cells in accordance with the same procedures as described in section 2 above. HUVECs were cultured until reaching 80-90% confluency in a 10 cm dish for adherent cells using a medium (hereinafter referred to as "EGM-2 medium") prepared from EBM^{™}-2 basal medium (CC-3156) and EGM^{™}-2 supplement kit, were dissociated with a 0.05 trypsin/EDTA solution, and were collected. The cells were suspended by adding EGM-2 medium. Cell number and viability were measured using Countess2 automatic cell counter and trypan blue, and the cell suspension concentration was adjusted to 25,000 cells/mL. The cells were seeded in a 96-well plate (100 µL/well). One day later, the medium was replaced with either CM or EGM-2 medium supplemented with the 50-100 kDa fraction. After culturing for 3 days, WST solution (10 µL/well) from Cell Counting Kit-8 was added, and after 3 to 4 hours of incubation, absorbance at 450 nm was measured.

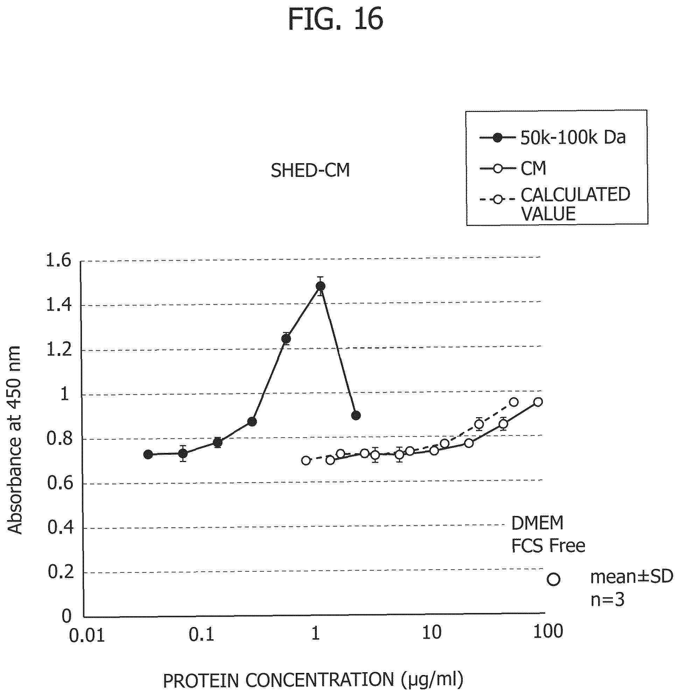

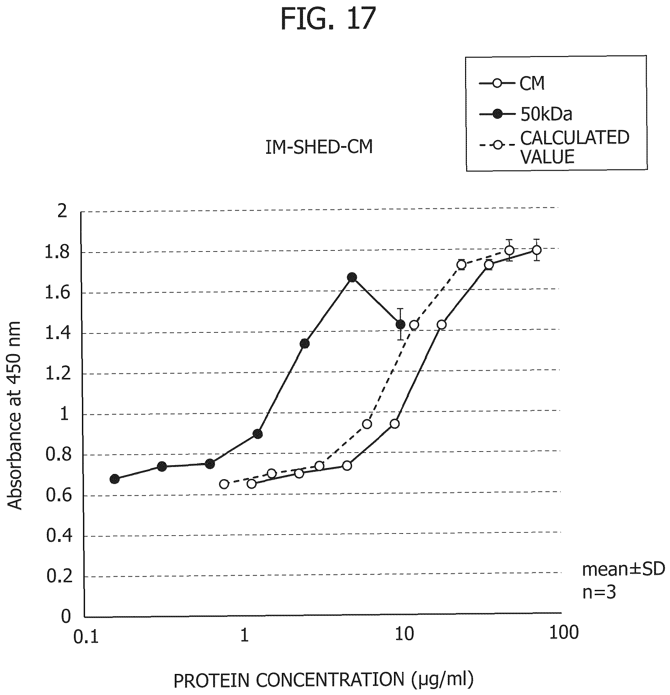

The results are shown in FIGs. 16 and 17. In the figures, "calculated value" indicates a value obtained by correcting the CM results based on the protein concentration of each fraction, which was determined from the electrophoresis results described in section 1 above and protein quantification using the Micro BCA Protein Assay Kit. Both 50-100 kDa fractions from SHED-CM and IM-SHED-CM showed greater intracellular dehydrogenase activity than CM at the same protein concentration. This result demonstrates that the 50-100 kDa fractions from SHED-CM and IM-SHED-CM contain component(s) that activate the proliferation and viability of HUVECs.

### <8. Inhibition of cell proliferation and viability by SHED-CM/IM-SHED-CM fraction - (2)>

A mixture of the >100 kDa, 50-100 kDa, and 30-50 kDa fractions (mixing ratio: 1:1:1) and a mixture of the 50-100 kDa and <30 kDa fractions (mixing ratio: 1:1) were prepared. The 50-100 kDa fraction and these mixtures were each serially diluted with EGM-2 medium and were evaluated for intracellular dehydrogenase activity by the same procedures as in section 7 above.

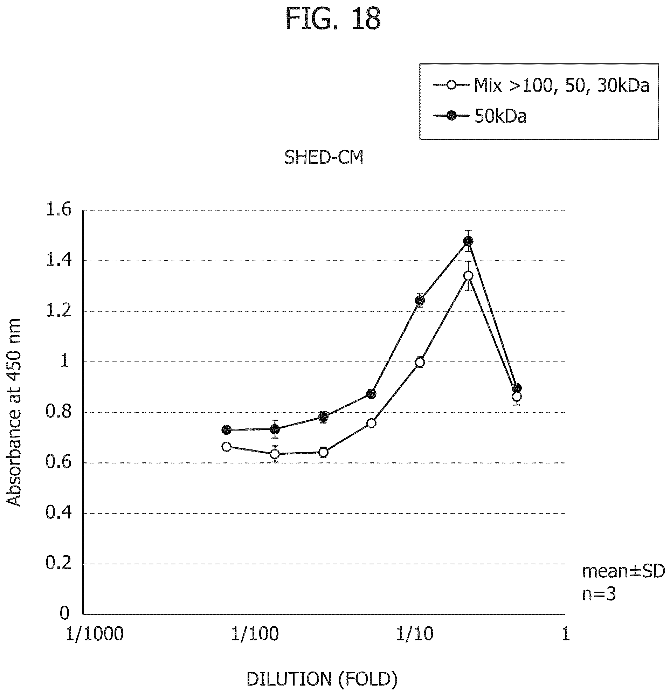

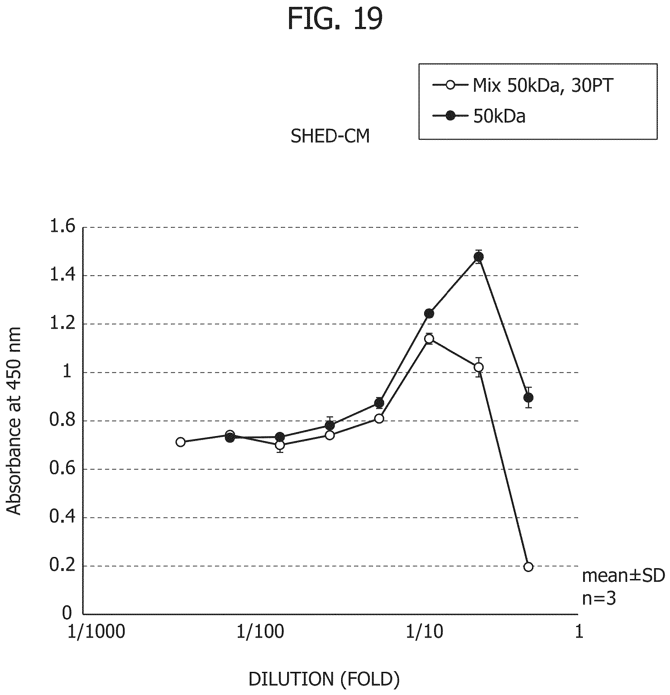

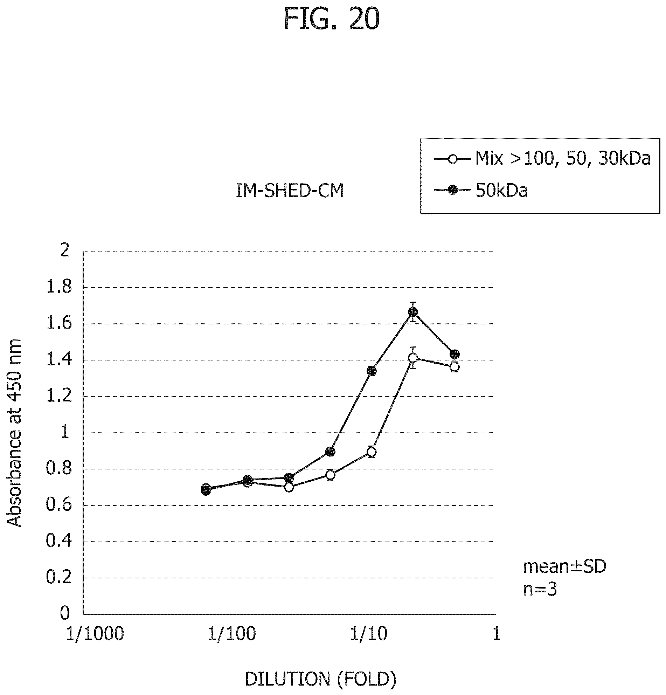

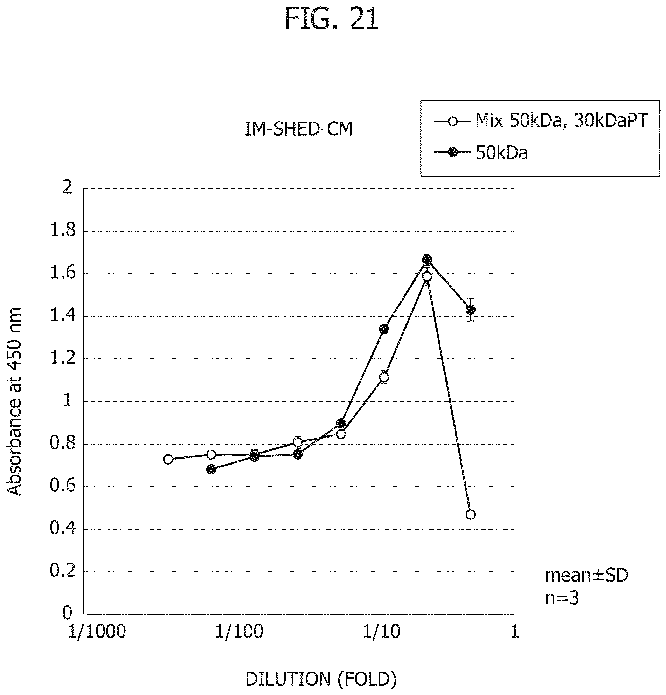

The results for the SHED-CM-derived fractions and mixtures are shown in FIGs. 18 and 20. The results for the IM-SHED-CM-derived fractions and mixtures are shown in FIGs. 19 and 21. In the figures, "Mix >100, 50, 30 kDa" denotes the mixture of the >100 kDa, 50-100 kDa, and 30-50 kDa fractions; "Mix 50 kDa, 30PT" denotes the mixture of the 50-100 kDa and <30 kDa fractions; and "50 kDa" denotes the 50-100 kDa fraction. The mixture of the >100 kDa, 50-100 kDa, and 30-50 kDa fractions showed no significant reduction in intracellular dehydrogenase activity compared with the 50-100 kDa fraction (FIGs. 18 and 20). In contrast, the mixture of the 50-100 kDa and <30 kDa fractions showed significant reduction in intracellular dehydrogenase activity compared with the 50-100 kDa fraction (FIGs. 19 and 21). These results demonstrate that component(s) of less than 30 kDa in CM inhibited proliferation and viability of HUVECs.

### <9. Suppressive effect of IM-SHED-CM fraction on hydrogen peroxide-induced cytotoxicity>

HUVECs were cultured until reaching 80-90% confluency in a 10 cm dish for adherent cells using EGM-2 medium, were dissociated with a 0.05 trypsin/EDTA solution, and were collected. The cells were suspended by adding EGM-2 medium. Cell number and viability were measured using Countess2 automatic cell counter and trypan blue, and the cell suspension concentration was adjusted to 50,000 cells/mL. The cells were seeded in a 96-well plate (100 µL/well). One day later, the medium was replaced with the serial dilutions of IM-SHED-CM or the fraction thereof with EGM-2 medium, and the cells were cultured for 3 days. Hydrogen peroxide (final concentration: 0.005 to 0.00031%) was added and the cells were incubated at 37°C for 1 hour. The dilutions were removed, EGM-2 medium (100 µL/well) and WST solution (10 µL/well) from Cell Counting Kit-8 were added, and after 3 to 4 hours of incubation, absorbance at 450 nm was measured.

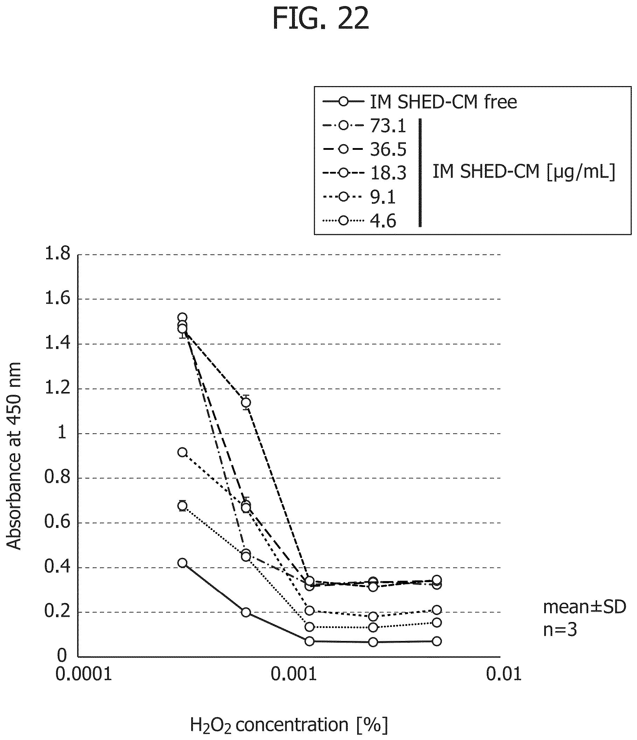

The results for the cells supplemented with the serial dilutions of IM-SHED-CM are shown in FIG. 22. In the cells not supplemented with IM-SHED-CM (control), absorbance at 450 nm decreased after hydrogen peroxide treatment and decreased further in a hydrogen peroxide concentration-dependent manner. These results confirmed cytotoxicity caused by hydrogen peroxide treatment. In contrast, in the cells supplemented with the serial dilutions of IM-SHED-CM, the decrease in absorbance at 450 nm was suppressed compared to the control, although absorbance at 450 nm decreased in a hydrogen peroxide concentration-dependent manner. These results demonstrate that IM-SHED-CM suppressed cytotoxicity caused by oxidative stress.

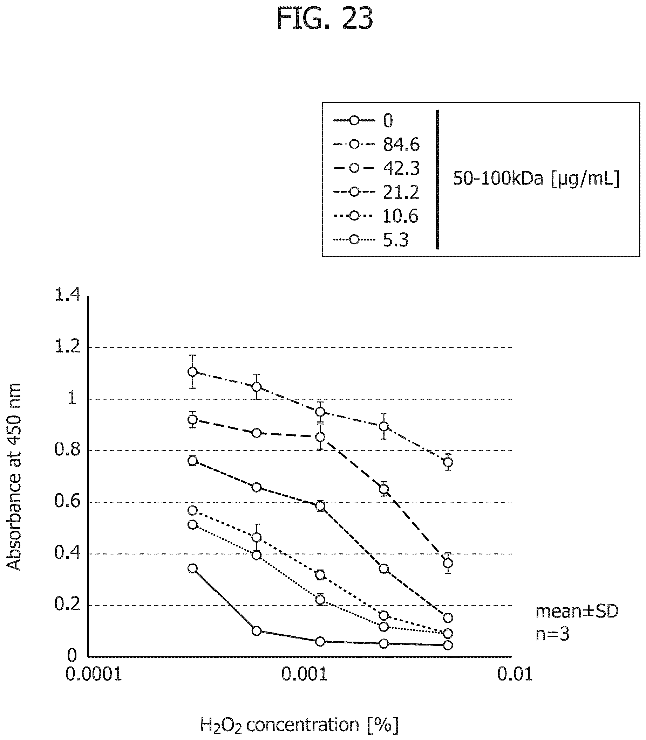

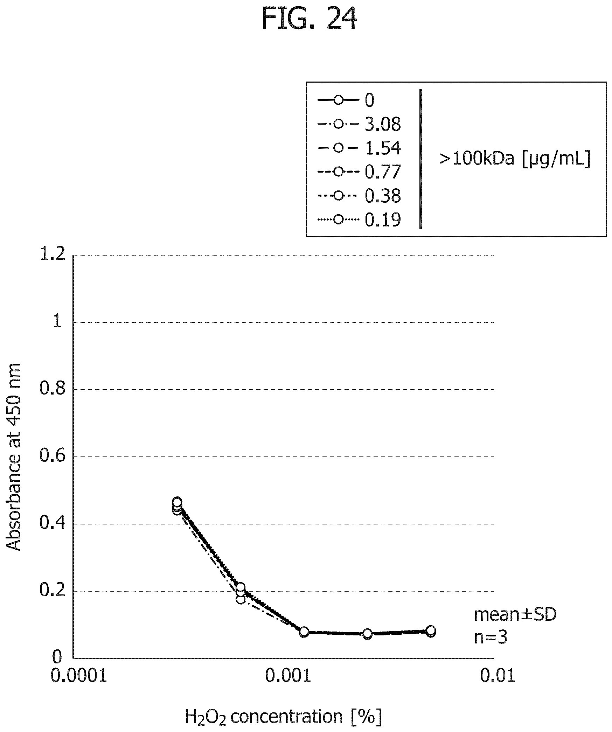

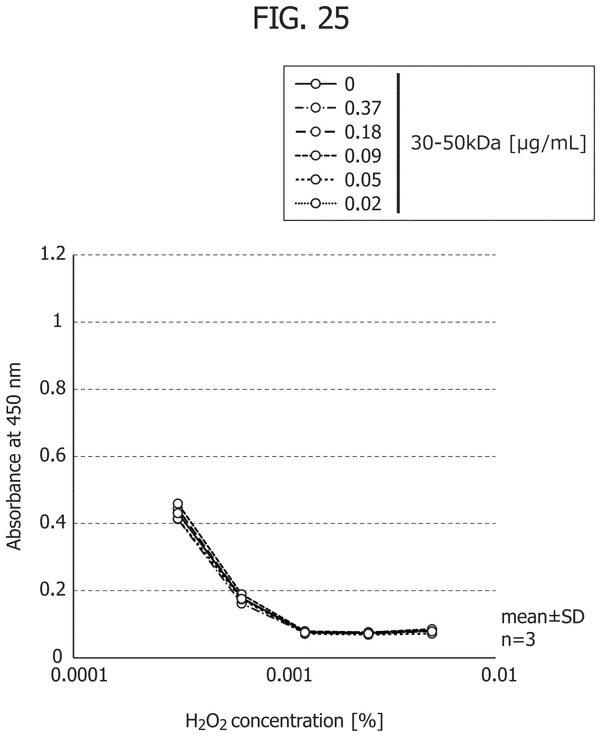

The results for the cells supplemented with the serial dilutions of the 50-100 kDa fraction of IM-SHED-CM are shown in FIG. 23. The results for the cells supplemented with the serial dilutions of the >100 kDa fraction are shown in FIG. 24. The results for the cells supplemented with the serial dilutions of the 30-50 kDa fraction are shown in FIG. 25. In the cells supplemented with the 50-100 kDa fraction, absorbance at 450 nm increased in a protein concentration-dependent manner, indicating that cytotoxicity due to oxidative stress was suppressed in a concentration-dependent manner by the 50-100 kDa fraction.

## Claims

1. A cell activator comprising a fraction isolated from a culture supernatant of a deciduous tooth pulp stem cell, wherein the fraction comprises no component of less than 3.5 kDa.

2. The cell activator according to claim 1, wherein the fraction comprises a component of 50 to 100 kDa and comprises no component of less than 30 kDa.

3. The cell activator according to claim 1 or 2, wherein the fraction comprises no component of more than 100 kDa and/or no component of 30 to 50 kDa.

4. The cell activator according to any one of claims 1 to 3, wherein the deciduous tooth pulp stem cell is a primary cultured cell.

5. The cell activator according to any one of claims 1 to 3, wherein the deciduous tooth pulp stem cell is an immortalized cell.

6. A method for activating a cell in a subject, comprising the step of administering the cell activator according to any one of claims 1 to 5 to the subject.

7. Use of a fraction isolated from a culture supernatant of a deciduous tooth pulp stem cell as a cell activator, wherein the fraction comprises no component of less than 3.5 kDa.

8. The use according to claim 7, wherein the fraction comprises a component of 50 to 100 kDa and comprises no component of less than 30 kDa.

9. The use according to claim 7 or 8, wherein the fraction comprises no component of more than 100 kDa and/or no component of 30 to 50 kDa.

10. The use according to any one of claims 7 to 9, wherein the deciduous tooth pulp stem cell is a primary cultured cell.

11. The use according to any one of claims 7 to 9, wherein the deciduous tooth pulp stem cell is an immortalized cell.
